# EUROPEAN PATENT SPECIFICATION

(11) **EP 2 981 226 B1**
(45) Date of publication and mention of the grant of the patent: **10.05.2017**
(21) Application number: 14714385.3
(22) Date of filing: 10.03.2014
(51) Int. Cl.: A61B 34/30, A61B 46/10

(54) **SURGICAL SYSTEM WITH STERILE WRAPPINGS**
CHIRURGISCHES SYSTEM MIT STERILEN UMHÜLLUNGEN
SYSTÈME CHIRURGICAL AVEC ENVELOPPES STÉRILES

(30) Priority: 05.04.2013 IT MI20130516
(43) Date of publication of application: 10.02.2016
(73) Proprietor: TransEnterix Italia S.r.l., 20154 Milano (MI) (IT)
(72) Inventor: GIORGI, Fabio, 16132 Genova (GE) (IT)
(74) Representative: Lampis, Marco
(86) International application number: PCT/IB2014/059577
(87) International publication number: WO 2014/162217

(56) References cited:
- WO-A1-2012/133912
- US-A- 5 413 573
- US-A- 6 142 937
- US-A1- 2004 049 205
- US-A1- 2010 198 220
- US-A1- 2011 277 775

## Description

The present invention relates to a surgical system, in particular for mini-invasive surgical operations, such as endoscopic operations, preferably but not exclusively of the robotic type.

It is known that in the prior art there exist surgical systems consisting of a first support element (for example a manipulator or a robot arm) onto which a surgical instrument, for example intended for mini-invasive surgical operations, is fixed.

For example, the surgical instrument may comprise a main body which is intended to be fixed into a suitable seat provided on the said first element and which has, projecting from it, an endoscopic tube which terminates in the active end of the instrument (forceps or the like). The main body houses internally the mechanical transmission systems and any electrical actuators and/or sensors of the instrument.

The surgical instrument thus designed is generally provided with a rapid release system so that it may be easily replaced inside the seat.

One problem affecting the known systems is that of ensuring the sterility of the operating environment. For example, a robot arm obviously cannot be sterilized as a whole and therefore, before a surgical operation, it is usually lined with a special plastic sleeve which has been sterilized beforehand.

In some cases the entire surgical instrument can be sterilized but often the fact that electronic and electromechanical components are contained inside the main body prevents sterilization, which is therefore carried out only on the endoscopic tube which for this purpose is designed to be detachable from the main body.

It is preferable, however, that the sterile sleeve should not extend over the instrument so as to avoid hindering replacement thereof during the operation. Replacement of the instrument is in fact indispensable for being able to switch between different types of instruments. For operation of the instrument mechanical coupling systems have been proposed for example where their two halves are coupled together so that they remain on the two sides of the sterile sheath or cover.

It would however be preferable for certain types of instruments to have electrical connections with the exterior for motorized operation of particular functions and/or for using sensors. This however conflicts with the need to ensure that a sterile barrier is maintained between the part provided with seat (for example the robot arm) and the surgical environment, in particular in the zone closest to the operating field, while allowing the possibility for rapid removal of the instrument.The two part form of claim 1 is based on US 5413573 A. The general object of the present invention is to provide a surgical system which allows rapid change-over of the surgical instruments, while nevertheless allowing a suitable sterility to be provided and maintained also in the case of electrical connections being present between instrument and seat receiving said instrument.

In view of this object the idea which has occurred according to the invention is that of providing a surgical system comprising a first support element with a seat which receives in a removable manner a surgical instrument for operational use thereof, characterized in that into the seat is present at least one first electrical connector which is intended for electrical connection with a complementary second electrical connector presents in the instrument when the instrument is received in the seat, and in that at least one of the two said connectors is designed to be movable on command between a first retracted rest position and a second advanced position for electrical connection together with the other connector and is provided with electrical contacts for engagement in the other connector which are suitable to cause perforation, during the movement between first and second positions, of at least one sterile sheath which is interposed between the first connector and second connector.

In order to illustrate more clearly the innovative principles of the present invention and its advantages compared to the prior art, an example of embodiment applying these principles will be described below with the aid of the accompanying drawings. In the drawings:
- Figure 1 shows a view of the zone for coupling together a first support element and a surgical instrument in a surgical system;
- Figure 2 shows another view of the coupling zone according to Figure 1 which shows schematically cross-sectioned an assembly for electrically connecting together instrument and seat;
- Figures 3 and 4 show schematic views of the electrical connection assembly in a disengaged condition and electrical engaged condition;
- Figure 5 shows a schematic side view of the system according to Figure 1 in an operating condition;
- Figure 6 shows a side view of the system according to Figure 1 during an intermediate stage of engagement/disengagement of a surgical instrument;
- Figures 7 shows a partial, enlarged and cross-sectioned view of a connector zone during an initial disengaging stage;
- Figure 8 shows a schematic perspective view of a robotized surgical system applying the principles of the present invention.

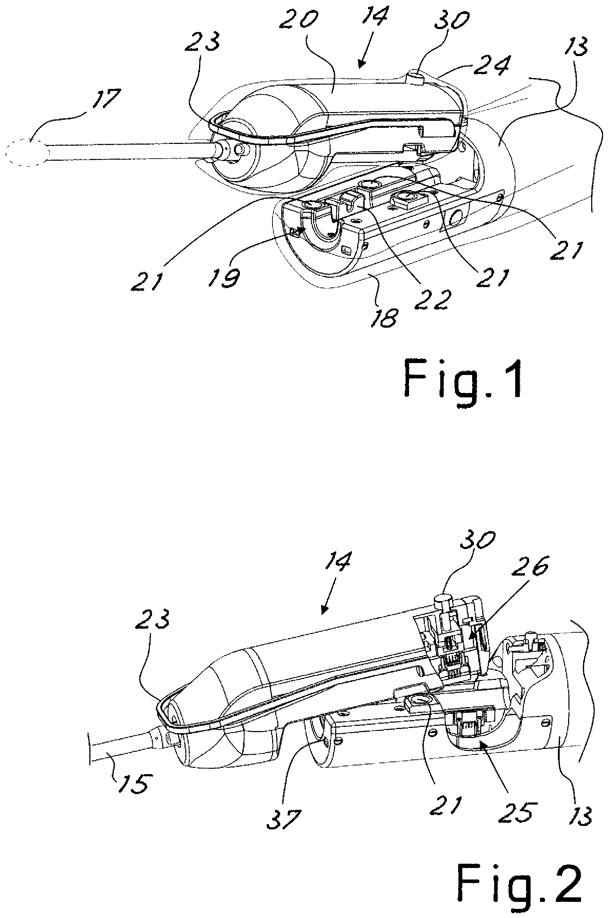

With reference to the figures, Figure 1 shows a zone for coupling together a first support element 13 and an instrument 14 in a surgical system.

This instrument will be, in general terms, for example a known instrument for performing endoscopic operations, with the end of the tube which terminates in the active end 17 of the instrument, such as a suitable surgical tool (for example fixed or directable forceps, an aspirator, a scalpel, etc.). In the case of forceps, the end could be for example articulated so as to rotate and/or swivel and these movements will be preferably controlled by electrical actuators arranged in the instrument body. One of the instruments may also advantageously comprise an endoscopic camera which records the operating field inside the patient.

The first element (of which only the end with the seat is shown for simpler illustration) may be for example a known manual manipulator for supporting and operating the instrument or a terminal zone or wrist zone of a robot arm, as will be clarified below.

For the sake of simplicity, in Figure 1 the active end 17 is shown as an oval form in broken lines, it being easy for the person skilled in the art to imagine many possible forms thereof.

As can be clearly seen in said Figure 1, the element 13 has a suitable seat 19 inside which the body 20 of the instrument 14 is removably fixed. A sterile sheath or cover 18, made of suitable thin plastic material (advantageously polyurethane), terminates in a closed end for enclosing the wrist and the seat 19. In this way the support element (for example a robot arm) and the seat 19 do not require sterilization.

The instrument 14 is engaged mechanically inside the seat with the arrangement of the sterile sheath 18 in between. For example, advantageously, the instrument may be fixed by means of a suitable magnetic retaining system 21 - known per se - which may comprise permanent magnets in the seat which attract complementary ferromagnetic plates on the instrument.

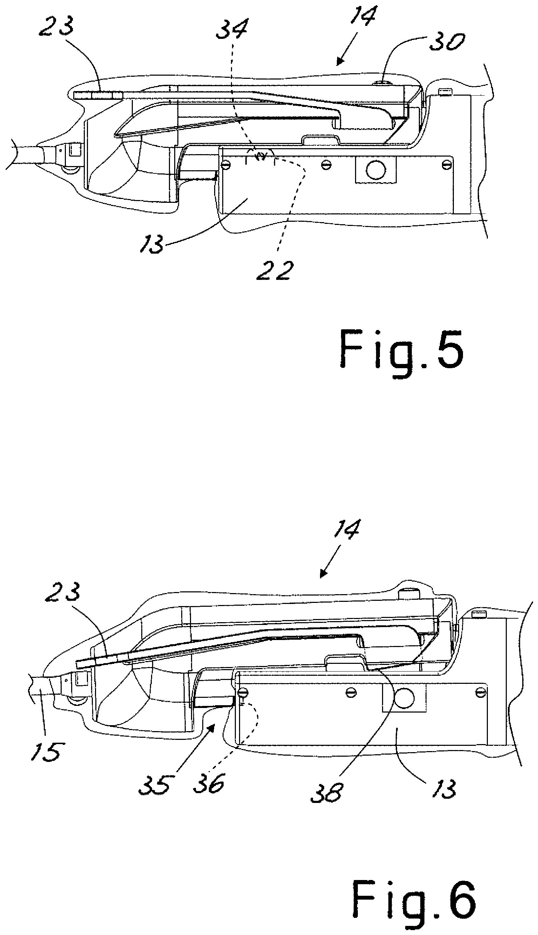

A known mechanical transmission system between seat and instrument body may also be present, still with the arrangement of the sterile sheath 18 in between. For example, it is possible to provide a fork-type coupling system 22 which is automated so as to slide parallel to the main axis of the instrument and which engages with a complementary mating element (for example in the form of a pin 34, shown schematically in broken lines in Figure 5) present on the side of the instrument which engages inside the seat, for transmitting a mechanical movement to the instrument. For example, in the case of a surgical tool in the form of forceps, the movement transmitted may be a movement for performing opening and closing of the forceps, so as to avoid the need to have, for this purpose, an electrical actuator in the instrument.

A handle 23 may be provided for facilitating removal of the instrument from the seat. This handle may also be of the lever type so that, when pulled or pushed, it acts with its opposite end 38 on the seat of the instrument in order to facilitate release of the instrument, as shown schematically in broken lines in Figure 6.

As can be seen again in Figure 1, if sterilization of the instrument is not possible or not desirable (for example because the instrument contains delicate electronic components), it may be advantageously inserted inside a second special sterile sheath or cover 24 which is made of flexible plastic (advantageously polyurethane) similar to that of the sheath 18 and from which preferably only the end which must make contact with the patient (for example the endoscopic tube) 15 protrudes, the latter being designed in this case so as to be disengageable in a known manner from the body 20, so that it may be sterilized using the known method.

The second sheath 24 is advantageously in the form of a bag which is open only at the front so that the instrument with the tube may be inserted therein and protrude from the opening. The opening is then sealed around the tube, for example using a suitable adhesive tape or an elastic band. The second sheath, where present, will therefore be also arranged between the instrument body and the seat 19.

As can be clearly seen in the partial cross-section of Figure 2 (where for the sake of simplicity the sheaths 18 and 24 are not shown) complementary electrical connectors 25, 26 are present on the seat and instrument body, said connectors being intended to engage with each other through the wall of the sterile sheath or sheaths, perforating the wall of the sheath which is clasped between the seat and instrument body. The connectors 25, 26 are connected internally to the respective electric circuits (not shown) which on one side extend inside the seat and on the other side are situated inside the instrument. These circuits may comprise sensors, actuators, control units, etc., as may be easily imagined by the person skilled in the art.

At least one of the two said connectors is designed to be movable controllably between a first retracted rest position and a second advanced position for electrical connection together with the other connector and is provided with electrical contacts for engagement in the other connector which are suitable for perforating, during the movement between a first and second position, the sterile sheath arranged so as to surround the said end of the first element with the seat and (if present) also the sterile sheath which surrounds the instrument and is arranged between the first and second connectors.

It has been found to be particularly advantageous if the movable connector is arranged on the instrument.

Advantageously, a manually operated control device 30 (preferably a pushbutton) mechanically controls the mutual engaging movement of the connectors. In this way, after the instrument has been arranged in position and fixed inside the seat, a manual pressure on the control device 30 causes the advancing movement of the contacts or pins of the movable connector 26 towards the complementary contacts or pins of the other connector 25, perforating the sterile sheaths and engaging the connectors together. Advantageously, the fact that perforation occurs at a point in the sheaths which is clasped between the seat and instrument body helps avoid tearing of the sheath at the perforation points and helps ensure that the holes are small and adhere to the side walls of the perforating pins. Sealing of the sterile barrier is thus ensured. Advantageously, the plastic of the sheaths may be chosen so as to be of a known type which is perforatable, but not easily torn.

The number, arrangement and size of the pins or contacts of the electrical connector will depend on the specific connection requirements of the instrument, as may be easily imagined by the person skilled in the art. For example, especially in the case of a relatively large number of pins, they may be arranged in several parallel rows.

As will be further described below, the advancing movement of the movable connector may take place against the action of spring means which exert a thrusting force for return towards the first rest position. Moreover, means are advantageously provided between the seat and the instrument for retaining the movable connector in the second position when the instrument is mounted inside the seat and the movable connector has been moved from the first position to the second position. In this way, by separating the instrument from the seat, the retaining means may also automatically release the movable connector which returns into its rest position. The retaining means may be advantageously of the magnetic type.

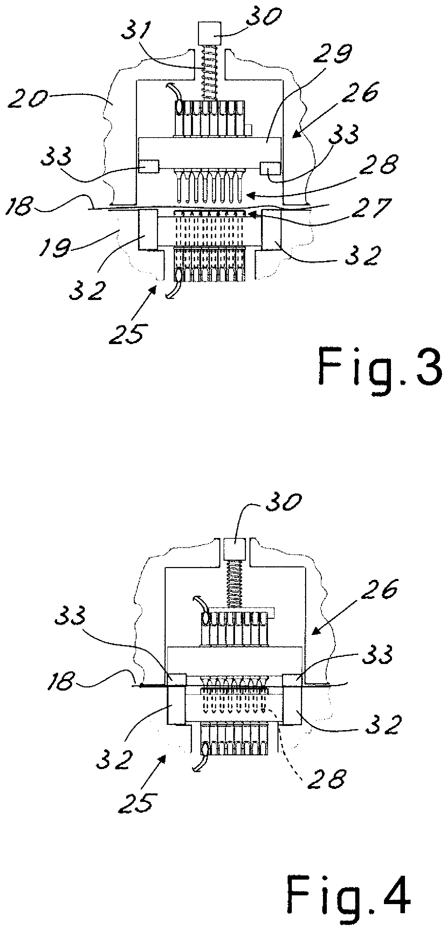

Figure 3 shows in schematic form an advantageous embodiment of the electric connectors 25, 26. In particular, the connector 25 is a substantially fixed connector with a certain number of electrical contacts or pins 27, advantageously of the female type, which extend inside the seat for engaging the instrument in the seat. The connector 26 instead comprises contacts or pins 28 which complement the contacts 27 and are mounted on a mobile support 29 so as to project from the support towards the contacts 27. The contacts 28 have an end with a tip suitable for perforating the wall of the sterile sheath. For example, they may be cylindrical with a conical tip. The tip may be sufficiently tapered and pointed so that it is able to perforate the sheet of the sheath without tearing it and, also, facilitate entry inside the female contacts. Advantageously the female contacts may have a flared (or funnel-shaped) inlet opening for facilitating entry/exit of the male pins also with a not perfectly axial movement. This allows, for example, an easy engagement and disengagement with a movement along a curved trajectory, as will be described below.

The support 29 may slide, against the action of springs (for example a spring 31 which acts underneath the pushbutton 30), between a rest position (shown in Figure 3), in which the contacts 28 are retracted, and an operating position (shown in Figure 4), in which the contacts 28 are advanced so as to project from the wall of the instrument until they perforate the sterile sheath and are inserted inside the corresponding contacts 27. Advantageously, in the rest position, the tips of the mobile contacts are completely retracted inside the instrument body so as to avoid possible distortion of the contacts and/or injury to the persons handling the instruments.

Once the connectors have been engaged, the connector 26 may remain with the contacts in the operating position owing to retaining means, until the instrument is disengaged from the seat for replacement. These retaining means may be, for example, mechanical means of a type known per se for releasable engagement of the support 26, such as pullpush systems (an initial pressure on the pushbutton engages them and a second pressure disengages them) or disengagement performed by the separation again of the instrument from the seat. Alternatively, the retaining means may also consist of the same contacts 27 and 28, if designed to have a suitable relative friction opposing accidental extraction. It has been found, however, to be particularly advantageous to use a magnetic retaining system which engages owing to the movement of the two connectors towards each other for engagement and which is released simply by the subsequent separation of the instrument from the seat. This system is particularly simple and very reliable.

As can be clearly seen in Figures 3 and 4, in order to provide such a magnetic system, advantageously magnets 32 are provided in one of the two connectors (preferably the connector present in the seat) and complementary ferromagnetic inserts 33 in the other connector. The relative position of the magnets and the inserts is such that, when the connectors are engaged by means of the pressure applied to the control device 30, the magnets and the inserts move toward each other sufficiently to attract each other through the sterile sheath and remain coupled together with a force such as to overcome that of the return spring 31. When the instrument body is raised away from the seat, the magnets and the inserts are separated and the movable connector may freely retract owing to the action of its return spring.

Figure 5 shows in schematic form the instrument 20 joined to its seat, with the connectors engaged together.

Advantageously, guide means are present between the instrument and seat and ensure (at least during a first disengaging movement) that a predetermined separation trajectory is followed so that the connectors may be extracted from each other without irreversibly twisting the contacts.

In particular, as schematically shown in Figure 6, guide means 35 may be provided for obliging the instrument to follow an initial curved trajectory with a radius of the trajectory followed by the contacts which is wide enough to allow extraction of the contacts without irreversible distortion.

Advantageously the guide means (for example consisting of pins 36 which engage inside seats 37 with suitable play) may be arranged towards the front of the body and seat, while the connector zone is close to the rear part thereof, such as to have a relatively large distance between the connectors and the point of rotation provided by the guide means 35 and therefore a sufficiently wide radius. As can be seen again in Figure 6, the release lever 23, where present, may have a thrusting end 38 which pushes against the bottom of the seat inside the seat so as to assist subsequent separation of the instrument from the seat. From a comparison of Figures 5 and 6 it can be understood how the lever may be operated so as to assist separation of the instrument from the seat.

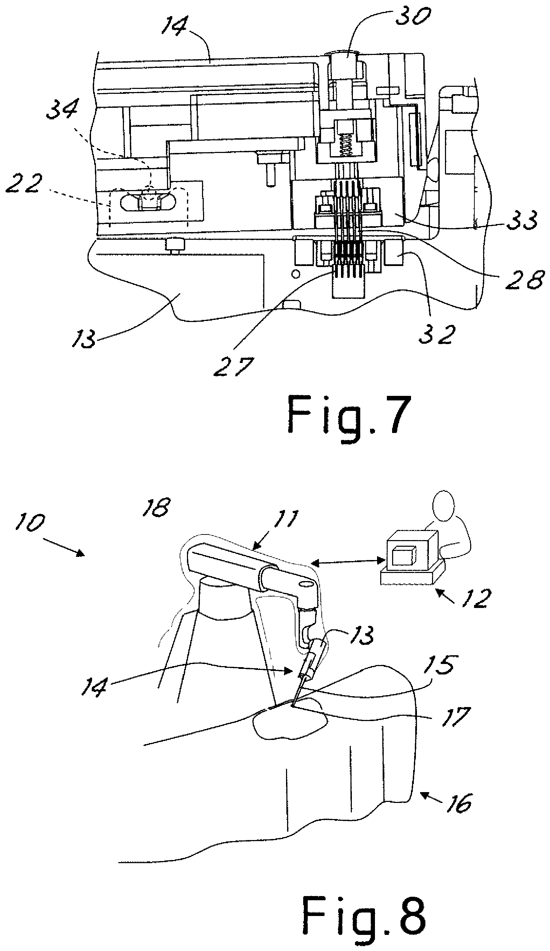

Figure 7 shows in schematic form an example of the initial extraction of the pins of the two connectors along a curved trajectory which does not permanently distort the contacts.

Figure 8 shows in schematic form an advantageous embodiment of the surgical system according to the invention, denoted generically by 10, in a robotized surgical application.

The system 10 comprises at least one robot arm 11 which operates under the control of a command console 12 manned by the surgeon.

The robot arm will be of the type substantially known and designed for the specific use. The surgical station may also comprise several robot arms even though here, for the sake of simplicity, only one is shown and described.

The robot arm (or each robot arm) terminates in a wrist portion 13 designed to support and operate a surgical instrument 14 so as to act by means of its end 15 (for example an endoscopic tube) on a patient 16. The robot arm receives the instrument inside a seat 19 in the wrist portion 13 which forms the said first positioning element for controllably positioning and directing the instrument in the operating space.

The robot arms, the instruments and the actuators for operating these instruments will not be further described or shown in the details, being known per se and easily imagined by the person skilled in the art. Also, the surgical operations which are possible with the system and their modes of preparation and execution are not further described here, being able to be easily imagined by the person skilled in the art.

The robot arm has, fitted thereon, a special sterile sheath or bag 18 which is suitably shaped so as to not to hinder the movement of the robot arm, as may be easily imagined by the person skilled in the art. This sheath 18 is intended to be perforated by the connector as described above, where applicable together with a second sheath placed around the instrument. The need to sterilize the entire robot arm is thus avoided.

At this point it is clear how the predefined objects have been achieved. Owing to the use of sterile sheaths, the wall of which is perforated only in the zone of the electrical connector by means of the contact movement of the connector pins, the sterile barrier is preserved. In the case also of replacement of the instrument, the contact zone already perforated does not alter substantially the sterility owing to the fact that the holes are small. Moreover, the material of the sterile sheath may be chosen with a suitable elasticity to obtain substantial re-closure of the holes when the perforating contacts are retracted.

With the solution described there is the further advantage that removal of the instrument is particularly rapid and does not require particular procedures, manual operation of a system for releasing the connector not being required for example.

Obviously the description provided above of an embodiment applying the innovative principles of the present invention is provided by way of example of these innovative principles and must therefore not be regarded as limiting the scope of the rights claimed herein. For example, the control device for movement of the connector may be different from a pushbutton (for example a lever) and/or comprise a mechanical transmission, also depending on the force required by the two connectors in order to perforate the sterile sheaths and be engaged together.

The surgical instrument may also be different from that described and not necessarily be designed for endoscopic use.

The male and female contacts, with any associated perforation/engaging mechanisms, may also be interchangeable, also depending on the utilisable space present on the part with seat and on the instrument. For example, in the case of a robot arm, there may be a limited amount of space such that advantageously the movement mechanism for perforation of the covers is provided on the instrument.

Moreover, the connectors may also be supported in an elastically pivoting manner so as to allow them to be adapted to the direction of insertion depending on the forces which arise between the male and female contacts during engagement or disengagement of the connectors.

Finally, further contactless signal transmission systems, for example of the optical, radio or electromagnetic induction type, may also be provided between instrument and seat.

## Claims

1. Surgical system comprising a first support element (13) with a seat (19) which receives in a removable manner a surgical instrument (14) for operational use thereof, **characterized in that** into the seat (19) is present at least one first electrical connector (25) which is intended for electrical connection with a complementary second electrical connector (26) presents in the instrument when the instrument is received in the seat, and **in that** at least one of the two said connectors (25, 26) is designed to be movable on command between a first retracted rest position and a second advanced position for electrical connection together with the other connector and is provided with electrical contacts for engagement in the other connector which are suitable to cause perforation, during the movement between first and second positions, of at least one sterile sheath (18) which is interposed between the first connector and second connector.

2. System according to Claim 1, **characterized in that** for the movement of the movable connector (26) from the first position to the second position it is provided a manually operated control command (30) acting against a spring (31) which is provided for returning the movable connector from the second position to the first position.

3. System according to Claim 1, **characterized in that** means (32, 33) are provided between the seat (19) and the instrument (14) for magnetically retaining the movable connector in the second position when the instrument is received in the seat and the movable connector has been moved from the first position to the second position.

4. System according to Claim 3, **characterized in that** the magnetic retaining means comprise permanent magnets (32) in one of the two connectors and ferromagnetic inserts (33) in the other of the two connectors for mutual attraction in their electrically connected condition.

5. System according to Claim 1, **characterized in that** a system (21) for magnetically retaining the instrument in the seat (19) is provided between said seat and the instrument (14).

6. System according to Claim 1, **characterized in that** the movable connector (26) is provided on the instrument and the fixed connector (25) is provided in the seat in a complementary position.

7. System according to Claim 1, **characterized in that** the movable connector (26) has male contacts (28) intended to be inserted inside complementary female contacts (27) in the other connector.

8. System according to Claim 1, **characterized in that** the instrument (14) comprises a body (20), which is intended to be received into the seat (19) and is provided with the second connector (26), and an endoscopic tube (15) projecting from the body (20) and terminating at the free end in an active end (17) of the instrument (17).

9. System according to Claim 8, **characterized in that** the sterile sheath which is interposed between the first connector and second connector comprises a sheath part arranged so as to surround at least partially the zone of the first element with the seat and/or a sheath part which surrounds the body (20) of the instrument.

10. System according to Claim 1, **characterized in that** guide means (35) are provided between the instrument and seat for a first separating movement of the instrument away from the seat along a predefined trajectory for relative extraction of the first and second connectors.

11. Surgical system according to any one of the preceding claims, **characterized in that** it comprises a robot arm (11) which terminates at its end in the said first support element (13) for positioning and directing in the space, by means of the robot arm, the instrument (14) mounted in the seat.

12. System according to Claim 11, **characterized in that** at least the end of the robot arm with the said first support element is surrounded by a sterile sheath (18) which covers the said seat (19) and is perforated by the electrical contacts of the said at least one connector (25, 26) when the instrument is received inside the seat and the movement of the said at least one connector towards its advanced position for electrical connection with the other connector is performed.

13. System according to any one of the preceding claims, **characterized in that** the sterile sheath is made of polyurethane at least along the part which is arranged between the first connector and second connector.

14. System according to any one of the preceding claims, **characterized in that** the electrical contacts designed to perform the perforation of the sheath are male contacts with a pointed end which are inserted inside female contacts in the other connector.

15. System according to Claim 14, **characterized in that** the female contacts have a tapered inlet opening for guiding the entry of the male contacts.

## Patentansprüche

1. Chirurgisches System, welches umfasst, ein erstes Trägerelement (13) mit einem Sitz (19), welcher in einer entfernbaren Art und Weise ein chirurgisches Instrument (14) zu dessen Betrieb aufnimmt, **dadurch gekennzeichnet, dass** in dem Sitz (19) mindestens ein erster elektrischer Verbinder (25) vorhanden ist, der, wenn das Instrument in dem Sitz aufgenommen wird, zur elektrischen Verbindung mit einem in dem Instrument vorhandenen komplementären zweiten elektrischen Verbinder (26) gedacht ist, und dass mindestens einer der beiden Verbinder (25, 26) ausgestaltet ist, auf Befehl zwischen einer ersten zurückgezogenen Ruhe-Position und einer zweiten vorgeschobenen Position zur elektrischen Verbindung mit dem anderen Verbinder bewegbar zu sein, und mit elektrischen Kontakten zum Eingriff in den anderen Verbinder versehen ist, die geeignet sind, während der Bewegung zwischen der ersten und zweiten Position, Perforationen in mindestens einer sterilen Lage (18) zu bewirken, die zwischen dem ersten Verbinder und zweiten Verbinder angeordnet ist.

2. System nach Anspruch 1, **dadurch gekennzeichnet, dass** für die Bewegung des bewegbaren Verbinders (26) von der ersten Position in die zweite Position eine manuell betriebene Steuerungseingabe (30) bereitgestellt ist, die gegen eine zur Rückführung des bewegbaren Verbinders von der zweiten Position in die ersten Position bereitgestellte Feder (31) wirkt.

3. System nach Anspruch 1, **dadurch gekennzeichnet, dass** zwischen dem Sitz (19) und dem Instrument (14) Mittel (32, 33) vorgesehen sind, um den bewegbaren Verbinder in der zweiten Position magnetisch zu halten, wenn das Instrument in dem Sitz aufgenommen wird und der bewegbare Verbinder von der ersten Position in die zweiten Position bewegt wurde.

4. System nach Anspruch 3, **dadurch gekennzeichnet, dass** das magnetische Rückhaltemittel zur gegenseiteigen Anziehung bei deren elektrischer Verbindung Permanentmagnete (32) in einer der beiden Verbinder und ferromagnetische Inserts (33) in dem anderen der beiden Verbinders aufweist,.

5. System nach Anspruch 1, **dadurch gekennzeichnet, dass** zwischen dem Sitz und dem Instrument (14) ein System (21) zum magnetischen Rückhalt des Instruments in dem Sitz (19) bereitgestellt wird.

6. System nach Anspruch 1, **dadurch gekennzeichnet, dass** der bewegbare Verbinder (26) an dem Instrument vorgesehen ist, und der fixierte Verbinder (25) in dem Sitz in einer komplementären Position bereit gestellt wird.

7. System nach Anspruch 1 , **dadurch gekennzeichnet, dass** der bewegbare Verbinder (26) vorstehende Kontakte (28) aufweist, die in die komplementären weiblichen Kontakte (27) in dem andere Verbinder inseriert werden sollen.

8. System nach Anspruch 1, **dadurch gekennzeichnet, dass** das Instrument (14) einen Körper (20) umfasst, der in dem Sitz (19) aufgenommen werden soll und mit dem zweiten Verbinder (26) versehen ist, und ein Endoskop-Rohr (15), das von dem Körper (20) vorsteht und an dem freien Ende in einem aktivem Ende (17) des Instruments (17) endet.

9. System nach Anspruch 8, **dadurch gekennzeichnet, dass** die sterile Lage, die zwischen dem ersten Verbinder und dem zweiten Verbinder angeordnet ist, ein Lagenteil umfasst, das angeordnet ist, um den Bereich des ersten Elements mit dem Sitz mindestens teilweise zu umgeben und/oder ein Lagenteil umfasst, das den Körper (20) des Instruments umgibt.

10. System nach Anspruch 1, **dadurch gekennzeichnet, dass** zwischen dem Instrument und dem Sitz für eine erste Trennbewegung des Instruments weg von dem Sitz entlang einer bestimmten Bahn zum Lösen des ersten und zweiten Verbinders Führnngsmittel (35) vorgesehen sind.

11. Chirurgisches System nach einem der vorstehenden Ansprüche, **dadurch gekennzeichnet, dass** es einen Roboterarm (11) umfasst, welcher an dessen Ende (13) zur Positionierung und Ausrichtung des in dem Sitz befestigten Instruments (14) im Raum mittels des Roboterarms in dem ersten Trägerelement endet.

12. System nach Anspruch 11, **dadurch gekennzeichnet, dass** mindestens das Ende des Roboterarms mit dem ersten Trägerelement von einer sterilen Lage (18) umgeben ist, welche den Sitz (19) bedeckt und welche durch die elektrischen Kontakte des mindestens einen Verbinders (25, 26) perforiert ist, wenn das Instrument in dem Sitz aufgenommen ist und der mindestens eine Verbinder auf eine vorgeschobene Position zur elektrischen Verbindung mit dem anderen Verbinder bewegt wird.

13. System nach einem der vorstehenden Ansprüche, **dadurch gekennzeichnet, dass** die sterile Lage mindestens entlang des Teils, welcher zwischen dem ersten Verbinder und dem zweiten Verbinder angeordnet ist, aus Polyurethan besteht.

14. System nach einem der vorstehenden Ansprüche, **dadurch gekennzeichnet, dass** die elektrischen Kontakte, die ausgestaltet sind, die Perforation der Lage zu bewirken, vorstehende Kontakte mit einem spitzen Ende sind, die in weibliche Kontakte in dem anderen Verbinder inseriert werden.

15. System nach Anspruch 14, **dadurch gekennzeichnet, dass** die weiblichen Kontakte eine sich verjüngende Einlassöffnung zum Einführen der männlichen Kontakte aufweisen.

## Revendications

1. Système chirurgical comprenant un premier élément de support (13) avec un siège (19) qui reçoit, de manière amovible, un instrument chirurgical (14) pour son utilisation opérationnelle, **caractérisé en ce que**, dans le siège (19), on trouve au moins un premier connecteur électrique (25) qui est prévu pour le raccordement électrique avec un second connecteur électrique complémentaire (26) présent dans l'instrument lorsque l'instrument est reçu dans le siège, et **en ce qu'**au moins l'un desdits deux connecteurs (25, 26) est conçu pour être mobile sur commande entre une première position de repos rétractée et une seconde position avancée pour le raccordement électrique conjointement avec l'autre connecteur et est prévu avec des contacts électriques pour la mise en prise dans l'autre connecteur, qui sont appropriés pour provoquer la perforation, pendant le mouvement entre les première et seconde positions, d'au moins une gaine stérile (18) qui est intercalée entre le premier connecteur et le second connecteur.

2. Système selon la revendication 1, **caractérisé en ce que**, pour le mouvement du connecteur mobile (26) de la première position à la seconde position, on prévoit une commande de contrôle actionnée manuellement (30) agissant contre un ressort (31) qui est prévu pour ramener le connecteur mobile de la seconde position à la première position.

3. Système selon la revendication 1, **caractérisé en ce que** l'on prévoit des moyens (32, 33) entre le siège (19) et l'instrument (14) pour retenir, par voie magnétique, le connecteur mobile dans la seconde position lorsque l'instrument est reçu dans le siège et que le connecteur mobile a été déplacé de la première position à la seconde position.

4. Système selon la revendication 3, **caractérisé en ce que** les moyens de retenue magnétiques comprennent des aimants permanents (32) dans l'un des deux connecteurs et des inserts ferromagnétiques (33) dans l'autre des deux connecteurs pour l'attraction mutuelle dans leur condition électriquement raccordée.

5. Système selon la revendication 1, **caractérisé en ce qu'**un système (21) pour retenir, par voie magnétique, l'instrument dans le siège (19), est prévu entre ledit siège et l'instrument (14).

6. Système selon la revendication 1, **caractérisé en ce que** le connecteur mobile (26) est prévu sur l'instrument et le connecteur fixe (25) est prévu dans le siège dans une position complémentaire.

7. Système selon la revendication 1, **caractérisé en ce que** le connecteur mobile (26) a des contacts mâles (28) prévus pour être insérés à l'intérieur des contacts femelles complémentaires (27) dans l'autre connecteur.

8. Système selon la revendication 1, **caractérisé en ce que** l'instrument (14) comprend un corps (20) qui est prévu pour être reçu dans le siège (19) et est prévu avec le second connecteur (26) et un tube d'endoscope (15) faisant saillie du corps (20) et se terminant au niveau de l'extrémité libre, par une extrémité active (17) de l'instrument (17).

9. Système selon la revendication 8, **caractérisé en ce que** la gaine stérile qui est intercalée entre le premier connecteur et le second connecteur comprend une partie de gaine agencée pour entourer au moins partiellement la zone du premier élément avec le siège et/ou une partie de gaine qui entoure le corps (20) de l'instrument.

10. Système selon la revendication 1, **caractérisé en ce que** l'on prévoit des moyens de guidage (35) entre l'instrument et le siège pour un premier mouvement de séparation de l'instrument à distance du siège le long d'une trajectoire prédéfinie pour l'extraction relative des premier et second connecteurs.

11. Système selon l'une quelconque des revendications précédentes, **caractérisé en ce qu'**il comprend un bras robotisé (11) qui se termine, au niveau de son extrémité, par ledit premier élément de support (13) pour positionner et rediriger dans l'espace, au moyen du bras robotisé, l'instrument (14) monté dans le siège.

12. Système selon la revendication 11, **caractérisé en ce qu'**au moins l'extrémité du bras robotisé avec le premier élément de support est entourée par une gaine stérile (18) qui recouvre ledit siège (19) et est perforée par les contacts électriques dudit au moins un connecteur (25, 26), lorsque l'instrument est reçu à l'intérieur du siège et que le mouvement dudit au moins un connecteur vers sa position avancée pour le raccordement électrique avec l'autre connecteur, est réalisé.

13. Système selon l'une quelconque des revendications précédentes, **caractérisé en ce que** la gaine stérile est réalisée à partir de polyuréthane au moins le long de la partie qui est agencée entre le premier connecteur et le second connecteur.

14. Système selon l'une quelconque des revendications précédentes, **caractérisé en ce que** les contacts électriques conçus pour réaliser la perforation de la gaine sont des contacts mâles avec une extrémité pointue, qui sont insérés à l'intérieur des contacts femelles dans l'autre connecteur.

15. Système selon la revendication 14, **caractérisé en ce que** les contacts femelles ont une entrée progressivement rétrécie s'ouvrant pour guider l'entrée des connecteurs mâles.
